# EUROPEAN PATENT SPECIFICATION

(11) **EP 4 066 827 B1**
(45) Date of publication and mention of the grant of the patent: **07.10.2026**
(21) Application number: 22160700.5
(22) Date of filing: 20.06.2017
(51) Int. Cl.: A61K 31/138, A61K 31/4245, A61K 31/436, A61K 31/519, A61P 35/00, A61P 35/04, A61P 43/00, C07D 413/12, C07D 413/10

(54) **AR+ BREAST CANCER TREATMENT METHODS**
VERFAHREN ZUR BEHANDLUNG VON AR+-BRUSTKREBS
MÉTHODES DE TRAITEMENT DU CANCER DU SEIN AR+

(30) Priority: 22.06.2016 US 201662353350 P; 19.08.2016 US 201662377497 P; 21.02.2017 US 201762461546 P
(43) Date of publication of application: 05.10.2022
(62) Document number(s) of the earlier application(s) in accordance with Art. 76 EPC: 17816081.8 / 3 474 841
(73) Proprietor: Ellipses Pharma Ltd, London, W1J 8LG (GB)
(72) Inventor: HATTERSLEY, Gary, Stow, 01775 (US); SAEH, Jamal, Belmont, 02478 (US); YU, Ziyang, Wellesley, 02481 (US); MILLER, Chris, Lake Forest, 60045 (US); BIHANI, Teeru, Cambridge, 02140 (US)
(74) Representative: Carpmaels & Ransford LLP

(56) References cited:
- US-A1- 2010 004 172
- US-A1- 2011 224 267
- US-A1- 2012 004 270
- US-A1- 2012 122 824
- US-A1- 2014 350 102
- US-A1- 2016 128 968
- US-A1- 2016 146 819
- COSS CHRISTOPHER C ET AL: "Selective androgen receptor modulators as improved androgen therapy for advanced breast cancer", STEROIDS, ELSEVIER SCIENCE PUBLISHERS, NEW YORK, NY, US, vol. 90, 16 June 2014 (2014-06-16), pages 94 - 100, XP029048267, ISSN: 0039-128X, DOI: 10.1016/J.STEROIDS.2014.06.010
- CHRIS P. MILLER ET AL: "Design, Synthesis, and Preclinical Characterization of the Selective Androgen Receptor Modulator (SARM) RAD140", ACS MEDICINAL CHEMISTRY LETTERS, vol. 2, no. 2, 2 December 2010 (2010-12-02), US, pages 124 - 129, XP055657906, ISSN: 1948-5875, DOI: 10.1021/ml1002508
- DUNNWALD LISA K ET AL: "Hormone receptor status, tumor characteristics, and prognosis: a prospective cohort of breast cancer patients", BREAST CANCER RESEARCH, CURRENT MEDICINE GROUP LTD, GB, vol. 9, no. 1, 19 January 2007 (2007-01-19), pages R6, XP021027025, ISSN: 1465-5411, DOI: 10.1186/BCR1639

## Description

### BACKGROUND

US2014/350102 and US2016/128968 concern the use of certain selective androgen receptor modulators in the treatment of certain breast cancers.

The relationship between androgens and breast cancer has been recognized for some time. In the past, androgen therapy has been used for treating breast cancer in women with mixed success. As breast cancer rapidly evolves to develop resistance to antiestrogen hormonal treatments, there is an urgent need to develop new treatment of breast cancer.

### SUMMARY OF THE INVENTION

The invention is set out in the appended set of claims. In particular, the invention provides a compound which is Compound III: 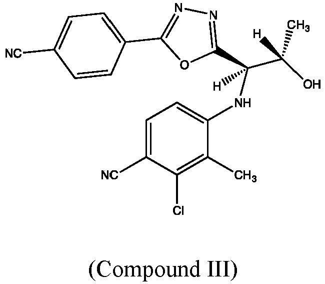 a pharmaceutically acceptable salt thereof, or a pharmaceutically acceptable solvate thereof, for use in a method of treating AR+/ER+/HER2- breast cancer in a subject.
Any references to methods of treatment are to be interpreted as references to compounds, pharmaceutical compositions and medicaments of the present invention for use in those methods.

Compound III is a selective androgen receptor modulator (SARM). In certain embodiments, the breast cancers comprise one or more mutations in the ER as disclosed herein. In certain embodiments, the one or more ER mutations affect the ability of the ligand binding domain of the ER to bind ligands having affinity to non-mutated ER (wild-type ER). In certain embodiments, the breast cancers initially positive for ER may lose tumor expression of ER. In certain embodiments, the subject is a premenopausal or postmenopausal woman. In certain embodiments, Compound III used for treating breast cancer is used in combination with a cell cyclin inhibitor and in some embodiments, the cell cyclin inhibitor is an inhibitor of CDK4 and/or CDK6 (CDK4/6 inhibitor). In certain embodiments, Compound III is used in combination with an mTOR inhibitor. In certain embodiments, the mTOR inhibitor is an inhibitor of mTOR2 and/or mTOR3. In some instances, Compound III is given by oral administration. In some embodiments, the cell cyclin inhibitor and/or mTOR inhibitor is given by oral administration. In certain embodiments Compound III and mTOR inhibitor or Compound III and CDK4/6 inhibitor are combined together in a kit. In some embodiments, Compound III and mTOR inhibitor or SARM and CDK4/6 inhibitor are coformulated.

In some embodiments of the methods disclosed herein, the cell cyclin inhibitor is a CDK4/CDK6 inhibitor selected from the group consisting of palbociclib, ribociclib, trilaciclib and abemaciclib. In certain embodiments, the CDK4/CDK6 inhibitor is a compounds that inhibits both CDK4 and CDK6 with an IC₅₀<250 nM or <100 nM or <50nM. In certain embodiments of the methods disclosed herein, the mTOR inhibitor (TORC1 and/or TORC2) is selected from the group consisting of sirolimus, temsirolimus, everolimus, ridafarolimus, and MLN0128. In certain embodiments, the methods of treating breast cancer using a combination of the SARM, Compound III, together with a PARP inhibitor and in some embodiments the PARP inhibitor is talazoparib, veliparib, niraparib, beigene290, E7449, KX01, ABT767, CK102, JPI289, KX02, IMP4297, SC10914, NT125, PJ34, VPI289, ANG-3186 are disclosed. In certain embodiments, the methods of treating breast cancer using a combination of Compound III together with a BCL2 inhibitor are described and in some embodiments the BCL-2 inhibitor is venetoclax, navitoclax, ABT737, G3139 or S55746. In certain embodiments, the methods of treating breast cancer using a combination of Compound III together with an MCL1 inhibitor are described and in some embodiments the MCL-1 inhibitor is 7-(5-((4-(4-(N,N-Dimethylsulfamoyl)piperazin-1-yl)phenoxy)methyl)-1,3-dimethyl-1H-pyrazol-4-yl)-1-(2-morpholinoethyl)-3-(3-(naphthalen-1-yloxy)propyl)-1H-indole-2-carboxylic Acid, S63845, omacataxine, seliciclib, UMI-77, AT101, sabutoclax, TW-37. In particular embodiments, the methods of treating breast cancer using a combination of Compound III together with a PI3K inhibitor.

In some embodiments of the methods disclosed herein, the breast cancer is treatment naive. In some embodiments, the breast cancer has not yet been treated with any endocrinological therapies. In certain embodiments of the methods disclosed herein, the breast cancer is resistant to at least one prior therapy. In some embodiments the prior treatment for which resistance has developed is an antiestrogen therapy, e.g., at least one of an aromatase inhibitor, a selective estrogen receptor modulator (SERM) or a selective estrogen receptor degrader (SERD). In certain embodiments, the subject (e.g., woman) is postmenopausal and has progressed on prior endocrine therapy, including, without limitation, SERDs (e.g., fulvestrant, RAD1901, AZD9496); SERMs (e.g., tamoxifen, toremifene), aromatase inhibitors, and combinations thereof. In some embodiments, the subject (e.g.woman) has metastatic breast cancer but has not yet been treated. In some embodiments, the subject (e.g.woman) has metastatic breast and has progressed after prior endocronological therapy. In some embodiments, the subject (e.g.woman) has metastatic breast and has progressed after treatment with a mTOR inhibitor, or a CDK4/6 inhibitor, or a PIK3 inhibitor.

A method of treating breast cancer described but not recited in the claims comprises the steps of measuring a baseline level of *ZBTB16* mRNA expression or protein expression in an ER+/AR+ breast cancer subject, treating with an AR agonist or selective androgen receptor modulator as described herein, measuring the level of *ZBTB16* (encoding protein PLZF) after the treatment, and if the *ZBTB16* level after treatment has increased, continuing the treatment with the AR agonist or selective androgen receptor modulator. In certain embodiments, the subject is a woman, e.g., premenopausal or postmenopausal woman.

Also described but not recited in the claims is a diagnostic kit containing reagents for measuring the mRNA or protein expression of *ZBTB16.*

Described herein but not recited in the claims is a method of identifying a subject who is likely to be responsive to the AR agonist therapy disclosed herein, comprising the steps of measuring a baseline level of *ZBTB16* mRNA expression or protein expression, treating with an AR agonist (e.g., SARM) for a period of time comprising at least one administration, measuring the *ZBTB16* mRNA expression level after the AR agonist therapy, and identifying the subject to be likely responsive to the AR agonist therapy if an increase in *ZBTB16* mRNA expression has occurred. In some embodiments, the expression cut off to determine responsiveness is at least a x2 fold increase, a x4 fold increase; an x8 fold increase, a x10 fold increase; a x25 fold increase; a x50 fold increase or a >1x100 fold increase.

In certain embodiments, a method of treating a woman with breast cancer is provided wherein said woman expresses one or mutations in the estrogen receptor, for example, a mutation of ERα gene (ESR1). Such mutations can include fusion proteins where part of the estrogen receptor has been fused to part or all of another protein. In some embodiments, a method of treating a woman with breast cancer is provided wherein said woman is first evaluated for one or more of said mutations and/or fusions and if she tests positive for the one or more mutations and/or fusions, she is treated with Compound III, either as a monotherapy or with one or more additional chemotherapeutics as described herein. In some embodiments, the subject expresses a mutated PI3K.

### BRIEF DESCRIPTION OF DRAWINGS

**Figure 1****:** Compound III ("RAD140") in combination with CDK inhibitor inhibited the growth of ER+/AR+ breast cancer in patient-derived xenograft (PDx) mice.
**Figure 2****:** Combined administration of RAD140 with CDK inhibitor or mTOR inhibitor inhibited the growth of ER+/AR+ breast cancer in cell line-derived xenograft (CDx) mice.
**Figure 3****:** RAD140 reduced the growth of ER+/AR+ breast cancer in PDx mice and was more efficacious than fulvestrant.
**Figure 4****:** RAD140 reduced the growth of ER+/AR+ breast cancer in PDx mice.
**Figures 5A-5B****:** Increase in *ZBTB16* mRNA expression in RAD140 treatment of breast cancer. Figure 5A: Increase in *ZBTB16* mRNA expression in RAD140 treatment of T47D breast cancer cells *in vitro.* Figure 5B: Increase in *ZBTB16* mRNA expression in RAD140 treatment of AR+/ER+ breast cancer *in vivo* (PDx #2).
**Figure 6****:** Inhibition effects of RAD140, palbociclib, and a combination of RAD140 and palbociclib on the PDx tumor in WHIM 18 models harboring the ESR1-Y AP1 fusion and an E545K mutation in PIK3CA.

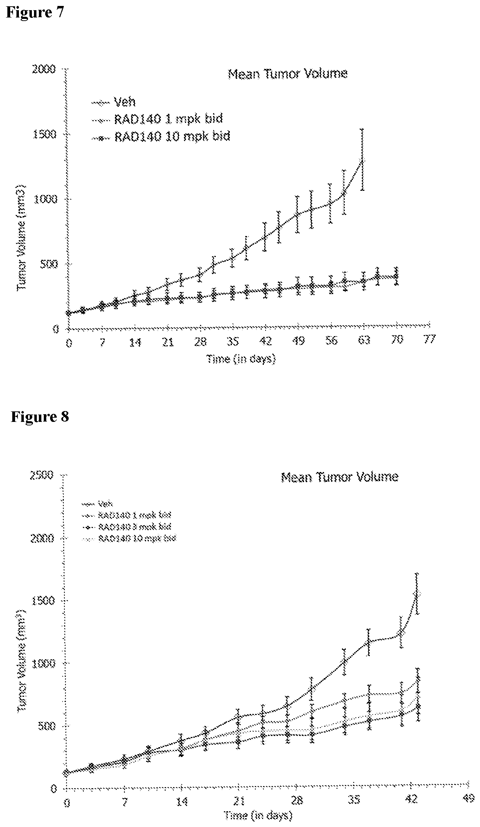

**Figure 7****:** RAD140 inhibited the growth of ER+/AR+ breast cancer patient-derived xenograft in the same PDX model as used in Figure 3.
**Figure 8****:** RAD140 inhibited the growth of ER+/AR+ breast cancer patient-derived xenograft in the same model as used in Figure 1.

### DETAILED DESCRIPTION OF THE INVENTION

RAD140 is an orally available, nonsteroidal SARM with a distinct tissue selectivity profile. In vitro functional analysis showed that RAD140 is a potent AR agonist, comparable to dihydrotestosterone in breast cancer cells. As set forth in the Examples section below, a treatment of a SARM (e.g., RAD140) alone (Examples 3 and 4; Examples 7 and 8) or in combination with a CDK4/6 inhibitor (e.g., palbociclib in Examples 1 and 2) or an mTOR inhibitor (e.g., everolimus in Example 2) effectively inhibited the growth of ER+/AR+ breast cancer in multiple PDx and/or CDx mice. Molecular analysis of the xenograft tumor specimen revealed a substantial suppression of progesterone receptor (PR), consistent with previous reports on the crosstalk between AR and ER pathways (ER signaling upregulates PR), but also demonstrated potent activation of the AR pathway in RAD140 treated tumors. Furthermore, increase in *ZBTB16* mRNA expression was observed in RAD140 treatment of AR+/ER+ breast cancer *in vivo* (PDx #2) and *in vitro* (T47D breast cancer cells); while PDx #2 treated with a SERD (fulvestrant) did not show appreciable induction of *ZBTB16* mRNA expression (Example 5). A treatment of a SARM (e.g., RAD140) alone (Example 6) or in combination with a CDK4/6 inhibitor (e.g., palbociclib) effectively inhibited the growth of ER+/PR+/AR+/HER- breast cancer in WHIM18 PDx mice initially expressing the ESR-1 YAP fusion.

More specifically, RAD140 unexpectedly inhibited tumor growth in all four PDx models (PDx #1 (AR+/ER+/PR+/Her2-), PDx #2 (AR+/ER+), PDx#3 (AR+/ER+), and WHIM18 PDx (ER+/PR+/AR+/HER-)) and CDx models (ZR75 CDx derived from ZR-75-1 cancer cell line (AR+/ER+) (See examples).

RAD140 alone showed tumor growth inhibition (TGI) to ER+/PR+/AR+/HER- breast cancer in WHIM18 PDx which was highly resistant to the potent ER-degrader fulvestrant (Example 6). Unexpectedly, administration of RAD140 in combination with a CDK4/6 inhibitor (e.g., palbociclib) resulted in enhanced TGI effects than treatment of RAD140 or palbociclib alone. Furthermore, in PDx #1 (Example 1) and CDx (Example 2) models, the administration of RAD140 in combination with a CDK4/6 inhibitor (e.g., palbociclib) again unexpectedly resulted in enhanced TGI effects compared to treatment of RAD140 or palbociclib alone. In CDx (Example 2) models, the administration of RAD140 in combination with a mTOR inhibitor (e.g., everolimus) also resulted in enhanced TGI effects compared with treatment of RAD140 or palbociclib alone (Example 2). Thus, SARMs (e.g., RAD140) are likely to be an effective endocrine backbone that potentiates the TGI of AR+/ER+ and/or ER+/PR+/AR+/HER- cancer treatment, including endocrine resistant or less responsive to endocrine therapy (e.g., SERDs such as fulvestrant) and also strongly potentiate the activities of effective agents such as a cdk4/6 inhibitor, an m-TOR inhibitor, PI3k inhibitors, PARP inhibitors, BCL-2 inhibitors, MCL-1 inhibitors, or any combinations thereof.

In certain embodiments, the methods further comprise administering to the subject a therapeutically effective amount of one or more second therapeutic agent(s) as described for herein, e.g., CDK4/6 inhibitors, mTOR inhibitors, PARP inhibitors, PIK3 inhibitors, BCL-2 inhibitors, and MCL-1 inhibitors.

Further described but not recited in the claims are methods for treating breast cancer in a subject comprising the following steps:
a) determining a baseline level of *ZBTB16* mRNA and/or protein;
b) administering an androgen receptor agonist;
c) treating the subject with the AR agonist and then retesting the level of *ZBTB16* mRNA and/or protein to provide a first level of *ZBTB16* mRNA and/or protein; and
d) continuing the administration of the AR agonist if the first level is higher than the baseline level of *ZBTB16* mRNA and/or protein.

Examples of the subjects include, without limitation, mammals, e.g., human. In certain embodiments, the subjects for the methods disclosed herein are women, e.g., premenopausal women as well as postmenopausal women. In certain embodiments, the subjects for the methods disclosed herein have progressed on prior endocrine therapy, including, without limitation, SERDs (e.g., fulvestrant, RAD1901, AZD9496); SERMs (e.g., tamoxifen, toremifene), aromatase inhibitors (e.g. arimidex, letrozole, aromasin or combinations thereof, whether direct or sequential). In some embodiments, the subjects have metastatic breast cancer and have progressed on prior endocrine therapy (e.g., SERDs, SERMs, aromatase inhibitors, or combinations thereof). In certain embodiments, the subject (e.g.woman) has primary or metastatic breast cancer and has progressed on a cdk4/6 inhibitor, an mTOR inhibitor, or a PI3K inhibitor. In some embodiments, the woman has not been treated for breast cancer and RAD140 is administered, either alone or in combination with one of the other agents mentioned herein.

The presence of AR, ER, and/or PR in breast cancer tumor cells or tumor tissue can be readily evaluated, e.g., by immunohistochemistry (IHC). Certain embodiments of the methods disclosed herein further comprise determining the tumor expresses AR and optionally one or more other receptors (e.g., ER, PR, and Her2), especially ESR1. Moreover, the methods disclosed herein open up the door to new treatment regimens not depending on pathways likely to already have been treated into resistance (e.g., antiestrogen resistance).

### Mutation of the ERa gene (ESR1)

Resistance to hormonal therapy in ER+ breast cancer is often accompanied by various mutations in the estrogen receptor. In some instances, these mutated receptors result in increased resistance or even complete resistance to anti-estrogen/anti-endocrinological treatment when such resistance comes as a result of treatment with an aromatase inhibitor, an antiestrogen SERM such as tamoxifen or an estrogen receptor degrader (SERD) such as fulvestrant. While in some instances, the resistance to anti-estrogen treatment is accompanied by complete loss of estrogen receptor signaling through loss of the receptor itself, however, in many instances the mutated receptor still signals through the ER pathway. The reasons for antiestrogen resistance include examples where the estrogen receptor is mutated in a way where it shows reduced affinity for directly competitive ligands (i.e. Y537S ESR1, D538G) or that it loses part or all of its ligand binding domain (LBD) but still retains enough of other functional domains (e.g. DNA binding domain, AF1 domain and/or the hinge region) so that the receptor even when unbound by ligand (or unable even to bind ligand) retains constitutive activity meaning that the receptor effectively remains switched on. In some instances this occurs where chromosomal translocations occur resulting in gene fusion products where the ligand binding domain of the ER is truncated or deleted and another gene or partial gene substituted in its place resulting in a constitutively active receptor that does not require ligand binding. It has been demonstrated that tumor cells harboring such genetic alterations are resistant to therapeutic agents targeting ER LBD. Such mutant cells can be enriched over time. Also, it is known that some of the mutations pre-exist treatment with antiestrogens/SERDs/aromatase inhibitors. A subject expressing such receptors may be at greater risk of poor response to conventional anti-ER treatment but are excellent candidates for AR agonist or SARM therapy as described herein. Thus a patient may be a candidate for first line treatment or adjuvant treatment AR agonist or SARM when the subject expresses such a mutation or fusion. In certain embodiments, a subject is tested for expression of a candidate mutation or fusion to determine whether to treat her in a neoadjuvant, adjuvant or first line setting where she has not yet been treated with an endocrinological agent. The first line use of the SARM can be either as a monotherapy or in combination with at least one agent selected from the group consisting of CDK inhibitors (e.g., CDK 4/6 inhibitors), mTOR inhibitors (e.g., mTORc 1 and/or 2 inhibitors), PARP inhibitors, PIK3 inhibitors, BCL-2 inhibitors and MCL-1 inhibitors. Prior to the herein described methods, these mutations were particularly problematic as they foreclose treatment with antiestrogenic agents which are first line for ER+ breast cancer patients. As a result, the patients often proceed to cytotoxic agents and experience a course of their disease that is often accelerated and much harder to treat. It is an important aspect of this disclosure that AR agonists described herein can effectively treat subjects harboring such mutated ER.

The AR agonists described herein may be used to treat subjects having AR+/ER+ breast cancer that comprises one or more ER mutations. In certain embodiments, these mutations affect the ability of the ligand binding domain to bind ligands having affinity to non-mutated ER. In certain embodiments of this invention, said ER has one or more point mutations in the ligand binding domain that reduce or eliminate binding to normally binding ER ligands of the type agonists and/or antagonists including SERMs and SERDs, and in some instances have constitutive ER signaling activity, e.g., resistant to aromatase inhibitors as well. In certain embodiments, the mutated receptor has a ligand binding domain that is partially or completely absent. In some embodiments, said mutant receptor is a fusion receptor between a part of ESR1 and part or all of another protein. In certain embodiments, said mutant receptor is capable of signaling through ER pathways despite not being able to bind ligand or having an attenuated affinity for ligands that bind non-mutated ER. In some embodiments, the mutant or fusion retains the ER of DNA-binding domain function.

In certain embodiments of the methods disclosed herein, the subject expresses at least one specifically described ER-fusion gene. In one embodiment, the gene comprises an E545K mutation product in PIK3CA, an ESR1-AKAP12 fusion product, an ESR1-CCDC170 gene fusion product, an ESR1-YAP1 gene fusion product, an ESR1-POLH gene fusion product, an ESR1-PCDH11X gene fusion product, or combinations thereof. In a particular embodiment, the subject expresses an ESR1-YAP1 fusion. In certain embodiments, the subject has his/her first breast cancers or tumors evaluated to determine if the subject harbor one or more ER gene-fusion mutations in one or more samples of their cancer cells. If the subject does indeed harbor one or more of the described ER mutations, the subject is a candidate for treatment according to the compounds and methods of this invention. In some embodiments of this invention, the subject has already been treated with one or more prior therapies and in some embodiments the said prior therapy comprises an antiestrogen therapy utilizing, for example, an aromatase inhibitor, a SERM or a SERD. In some embodiments, the subject with the indicated mutation has not been pretreated at all for his/her breast cancer or has not been pre-treated with an antiestrogen. In certain embodiments, the subject has one or more of the mutations/fusions compromising ligand binding function or ESR1 activity.

In certain embodiments, the subject is initially positive for ER and then loses tumor expression of ER. In some embodiments, said lost expression occurred after the course of one or more prior treatments. In certain embodiments, said one or more prior treatments comprised treatment with an antiestrogen further comprising one or more of an aromatase inhibitor, a SERM and a SERD. In certain embodiments, said subject who has lost ER expression in the tumor does express the progesterone receptor (PR).

In certain embodiments of the methods disclosed herein, the method disclosed herein further comprises a diagnostic step wherein said subject is first evaluated for one or more mutations/fusions as described herein. If the mutation/fusion is deemed to meet a predetermined cut off, the subject is a candidate for AR agonist/SARM treatment, either as a monotherapy or as a combination as described herein. In some embodiments, the mutant or fusion includes the ESR1-YAP fusion and/or closely related embodiments.

### AR agonists

AR agonists include steroidal AR agonist such as testosterone (and esters thereof), DHT (and esters thereof), fluoxymesterone, oxandrolone, stanzolol, methandrostenelone, methyltestosterone, oxymetholone, nandrolone (and esters thereof). In the invention, the AR agonist is the SARM RAD140, also referred to as Compound III. In certain embodiments, SARMs demonstrate efficacy on tumor endpoints despite having reduced androgen drive on other tissues (e.g., prostate) or other expression profiles resulting in undesired outcomes such as virilization and hirsutism in females. In certain embodiments, SARMs often present with reduced drive on liver enzymes elevations and/or possibly deleterious changes in cholesterol levels such as decreased HDl and/or increased LDL. In certain embodiments, SARMs are non-steroidal and do not present the potential class liability of 17alpha alkylated steroids though they still have good oral activity in general. In certain embodiments, SARMs provide effective treatments that are less or non-virilizing. In certain embodiments, SARMs are not likely to feedback stimulate the central hormonal axis. In certain embodiments, SARMs (e.g., RAD140) can cross the blood brain barrier (the "BBB"). In certain embodiments, SARMs that can cross the BBB have suppressive effects on the central hormonal axis and decrease rather than increasing the ovarian production of sex steroids, e.g., estrogens such as estrone and estradiol as well as intracrine precursors such as DHEA, androstenedione, etc. In certain embodiments of the methods disclosed herein, the SARMs may be generally used for treating premenopausal women with breast cancer as well as postmenopausal women with breast cancer. In certain embodiments, suppressive SARMs (e.g., RAD140) provide additional CNS benefits in premenopausal or postmenopausal women having breast cancer. In certain embodiments, the AR agonists (e.g., SARMs) disclosed herein increase lean muscle mass and/or appetite. Thus, the AR agonists (e.g., SARMs) disclosed herein can be beneficial where a cancer cachexic state or wasting is a concern.

SARMs include 2-chloro-4-[[(1*R*,2*R*)-2-hydroxy-2-methyl-cyclopentyl]amino]-3-methyl-benzonitrile (J Med Chem 2016; 59(2) 750), PF-06260414, enobosarm, BMS-564929, LGD-4033, AC-262356, JNJ-28330835, S-40503, GSK-2881078, RAD140, AZD-3514, MK4541, LG121071, GLPG0492, NEP28, YK11, MK0773, ACP-105, LY-2452473, S-101479, S-40542, S-42, LGD-3303 and the SARMs disclosed in US8,067,448 and US9,133,182. In addition, SARMs include compounds according to Formula I disclosed herein (e.g., Compound II and Compound III), and compounds according to Formula IV disclosed herein, which may be used alone or in combination with one or more agents selected from the group consisting of CDK inhibitors (e.g. CDK4/6 inhibitors), mTOR inhibitors (e.g., mTORc 1 inhibitors and/or mTORc 2 inhibitors), PARP inhibitors, PIK3 inhibitors, BCL-2 inhibitors, MCL-1 inhibitors, and combinations thereof, for the treatment of AR+ breast cancer in a subject.

Compounds according to Formula I include compounds having the structure of Formula I : 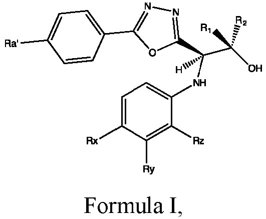 pharmaceutically acceptable salts thereof, and pharmaceutically acceptable solvates thereof, wherein:
Rₓ = CN;
R_{y} = CF₃ or Cl;
R_{z} = CH₃, CH₂CH₃ or Cl; or
R_{y} and R_{z} together form: 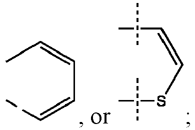
R_{a'} is H, F, Cl, CN, OH or OSO₃; and
R₁ and R₂ are each independently selected from hydrogen and methyl.

Compounds according to Formula I include Compound II (described herein but not recited in the claims) and Compound III (RAD140, recited in the appended claims): 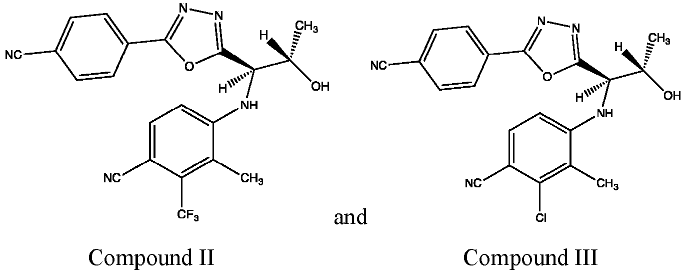 a pharmaceutically acceptable salt thereof, or pharmaceutically acceptable solvate thereof.

Compounds according to Formula IV described herein but not recited in the claims include: 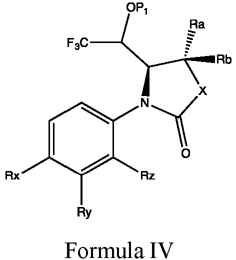 pharmaceutically acceptable salts thereof, and pharmaceutically acceptable solvates thereof, wherein:
Rₓ is CN, Cl, Br, or NO₂;
R_{y} is CH₃, CF₃, or halogen;
R_{z} is hydrogen, optionally substituted C₁₋₃ alkyl, optionally substituted C₂₋₃ alkenyl, optionally substituted C₁₋₃ hydroxyalkyl, optionally substituted C₁₋₃ haloalkyl, NO₂, NH₂, OMe, halogen or OH;
P₁ is hydrogen or a metabolically labile group;
Rₐ and R_{b} are each independently hydrogen or C₁₋₃ alkyl; and
X is O.

Examples of optional substitution include, without limitation, 1-3 halogen atoms.

An example of a SARM is a compound according to Formula IV, a pharmaceutically acceptable salt thereof, or pharmaceutically acceptable solvate thereof, wherein Rₓ is CN.

An example of a SARM is a compound according to Formula IV, a pharmaceutically acceptable salt thereof, or pharmaceutically acceptable solvate thereof, wherein Rₓ is CN; R_{y} is Cl or CF₃; R_{z} is hydrogen, Cl or CH₃; P₁ is (C=O)-C₁₋₆ alkyl or hydrogen; and Rₐ and R_{b} are each independently hydrogen or -CH₃.

An example of a SARM is a compound according to Formula IV, a pharmaceutically acceptable salt thereof, or pharmaceutically acceptable solvate thereof, wherein Rₓ is CN; R_{y} is Cl or CF₃; R_{z} is hydrogen, Cl or CH₃; P₁ is (C=O)-C₁₋₆ alkyl or hydrogen; and Rₐ and R_{b} are both hydrogen.

### Combination therapy of AR agonists and mTOR inhibitors

The phosphoinositide 3-kinase (PI3K)/protein kinase B (AKT)/mammalian target of rapamycin (mTOR) pathway is an intracellular signaling pathway important in regulating the cell cycle. The frequent activation of the PI3K/AKT/mTOR pathway in cancer and its crucial role in cell growth and survival provide a challenge in finding an appropriate amount of proliferation versus differentiation in order to utilize this balance in the development of various therapies. See, e.g., Gitto et al., "Recent insights into the pathophysiology of mTOR pathway dysregulation," Res. Rep. Bio., 2:1-16 (2015).

Inhibitors of the PI3K pathway have shown promises when given in combination with other therapies. For example, everolimus, as an allosteric mTOR inhibitor, was the first mTOR inhibitor approved in combination with AI exemestane (aromasin), for postmenopausal women with advanced hormone receptor positive (HR+), HER2- breast cancer (BOLERO-2 study) in 2012. Agents targeting other components of the PI3K pathway are under development for treating HR+ cancer, e.g., ATP-competitive, dual inhibitors of PI3K and mTOR (e.g., BEZ235, GDC-0980), pan-PI3K inhibitors which inhibit all four isoforms of class I PI3K (e.g., BKM120, GDC-0941), isoform-specific inhibitors of the various PI3K isoforms (e.g., BYL719, GDC-0032), allosteric and catalytic inhibitors of AKT (MK2206, GDC-0068, GSK2110183, GSK2141795, AZD5363), and ATP-competitive inhibitors of mTOR only (AZD2014, MLN0128, and CC-223). Dienstmann et al., "Picking the point of inhibition: a comparative review of PI3K/AKT/mTOR pathway inhibitors," Mol. Cancer Ther., 13(5):1021-31 (2014).

Despite their great potential, undesirable side effects associated with mTOR inhibitors have hindered their development as effective cancer therapies. Kaplan et al., "Strategies for the management of adverse events associated with mTOR inhibitors," Transplant Rev (Orlando), 28(3): 126-133 (2014); and Pallet et al., "Adverse events associated with mTOR inhibitors," Expert Opin. Drug Saf. 12(2): 177-186 (2013).

Furthermore, there remains a need for more durable and effective targeted therapies that can overcome challenges associated with the current endocrine therapies, while providing additional benefits by combining with a second therapeutic agents (e.g., everolimus and other agents targeting the PI3K/AKT/mTOR pathway) to combat cancer in advanced stage and/or with resistance to prior treatments.

In some embodiments, the second therapeutic agent targets the PI3K/AKT/mTOR pathway and can be a mTOR inhibitor, a dual mTOR inhibitor, a PI3K/mTOR inhibitor, or an inhibitor of mTOR2 and/or mTOR3. In some embodiments, the second therapeutic agent is a rapamycin derivative (aka rapalog) such as rapamycin (sirolimus or rapamune, Pfizer), everolimus (Affinitor or RAD001, Novartis), ridaforolimus (AP23573 or MK-8669, Merck and ARIAD Pharmaceuticals), temsirolimus (Torisel or CCI779, Pfizer), including solvates (e.g., hydrates) and salts thereof. In some embodiments, the second therapeutic agent is a dual mTOR inhibitor that inhibits both mTORC1 and mTORC2, such as MLN0128, CC115 and CC223 (Celgene), OSI-027 (OSI Pharmaceuticals), and AZD8055 and AZD2014 (AstraZeneca), including solvates (e.g., hydrates) and salts thereof. In some embodiments, the second therapeutic agent is a PI3K/mTOR inhibitor such as GDC-0980, SAR245409 (XL765), LY3023414 (Eli Lilly), NVP-BEZ235 (Novartis), NVP-BGT226 (Novartis), SF1126, and PKI-587 (Pfizer), including solvates (e.g., hydrates) and salts thereof.

In certain embodiments, more than one of the second therapeutic agents disclosed above may be used in combination with Compound III. For example, an mTOR inhibitor can be used together with another mTOR inhibitor or with a PI3K/mTOR inhibitor. Also, it is known in the art that the second therapeutic agents disclosed above, including mTOR inhibitors, dual mTOR inhibitors, and PI3K/mTOR inhibitors, can be administered with other active agents to enhance the efficacy of the treatment for example can be used in combination with JAK2 inhibitors (Bogani et al., PLOS One, 8(1): e54826 (2013)), chemotherapy agents (Yardley, Breast Cancer (Auckl) 7: 7-22 (2013)). Accordingly, the second therapeutic agents also include these auxiliary active agents.

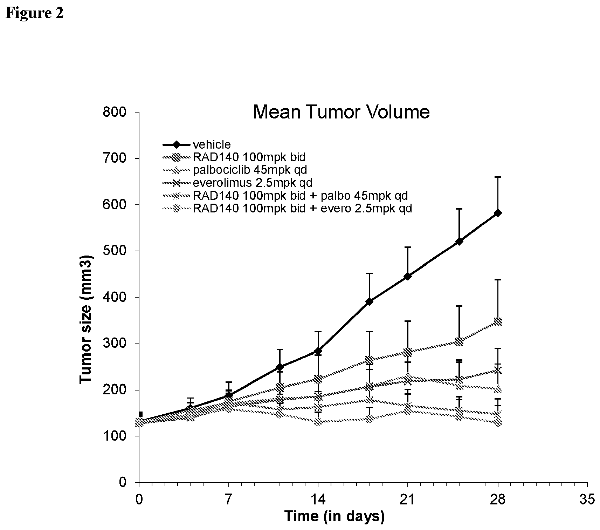

Figure 2 illustrates the enhanced efficacy obtained when a mTOR inhibitor everolimus was used in combination with a SARM RAD140 in an *in vivo* model

### Combination therapy of AR agonists and CDK inhibitors

Cell cycle regulators such as cyclins and cyclin-dependent kinases (CDKs) have been reported to have effects on ER expression. Lamb et al., "Cell cycle regulators cyclin D1 and CDK4/6 have estrogen receptor-dependent divergent functions in breast cancer migration and stem cell-like activity," Cell Cycle 12(15): 2384-2394 (2013). Selective CDK4/6 inhibitors (e.g., ribociclib, abemaciclib and palbociclib) have enabled tumor types in which CDK4/6 has a pivotal role in the G1-to-S-phase cell cycle transition to be targeted with improved effectiveness and fewer adverse effects to normal cells. O'Leary et al., "Treating cancer with selective CDK4/6 inhibitors," Nat. Rev. Clin. Oncol. (2016), published online 31 March 2016 (http://www.nature.com/nrclinonc/journal/vaop/ncurrent/full/nrclinonc. 2016.26html). The selective CDK4/6 inhibitors demonstrated the best responses when tested in combination with ER-endocrine therapy in patients with ER-positive breast cancer. In this regard, it is helpful to keep in mind that AR pathways can intersect with ER pathways and possibly under some conditions, antagonize ER activity in a different manner than direct ER antagonism.

Palbociclib in combination with the aromatase inhibitor letrozole (PALoMA-1/TRIO 18 study) was approved for the treatment of hormone receptor (HR)-positive (HR+), HER2-negative (HER2-) advanced breast cancer as initial endocrine based therapy in postmenopausal women in February 2015. In February 2016, palbociclib in combination with the SERD fulvestrant (PALOMA-3 study) was approved for the treatment of ER+, HER2- advanced or metastatic breast cancer patients that had progressed on prior endocrine therapy. The FDA has granted the CDK4/6 inhibitor abemaciclib (LY2835219) a breakthrough therapy designation as monotherapy for heavily pretreated patients with refractory HR-positive advanced breast cancer, based on data from a phase I study (JPBA study). Additional combinations of selective CDK4/6 inhibitors (e.g., ribociclib, abemaciclib and palbociclib) with ER- endocrine therapies (e.g., AIs, SERMs and SERDs) are currently under development. However, it appears clinical or preclinical studies demonstrating the unexpected combined efficacy between SARMs and cdk4/6 inhibitors appear lacking until now.

Furthermore, CDK4/6 inhibitors demonstrate toxicities that may require intermittent therapy (O'Leary). Thus, there remains a need for effective and alternative combinationtargeted therapies that can overcome challenges associated with the current endocrine therapies, while providing additional benefits by combining with CDK4/6 inhibitors to combat breast cancer, particularly in advanced stage and/or with resistance to prior treatments or with mutations identified as existing pre-prior therapy or after prior therapy that render the ESR1 target amenable to androgen intervention (including SARMs) where other endocrinological therapies are generally less effective or not effective at all, particularly those relying on a direct antiestrogenic effect such as an aromatase inhibitor, SERM or SERD.

In certain embodiments, the CDK4 and/or CDK6 inhibitors include, without limitation, palbociclib, abemaciclib, ribociclib and AMG925.

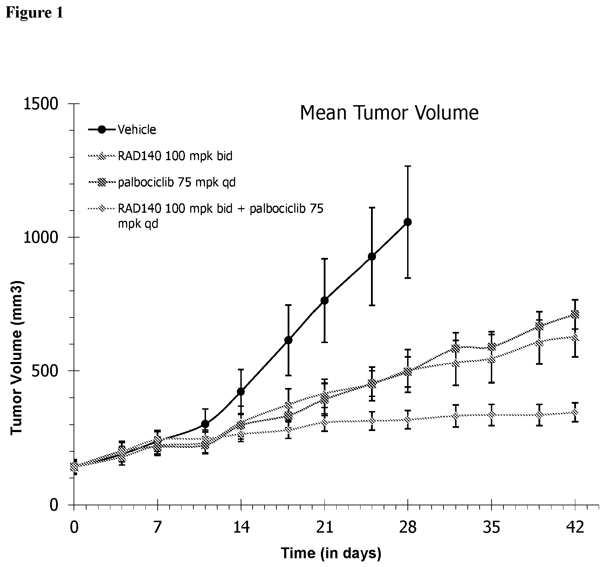

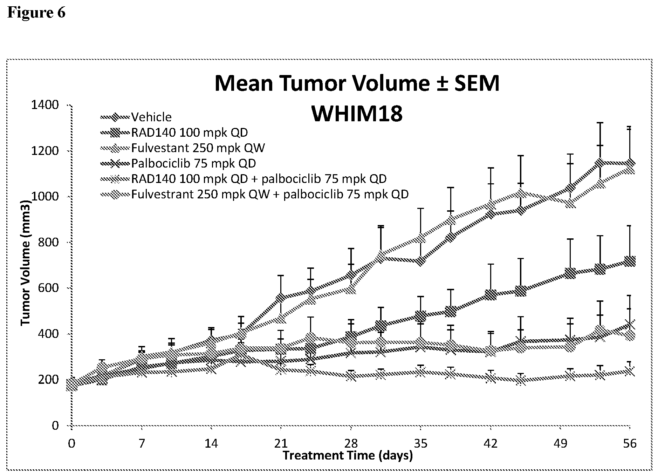

The *in vivo* illustration of this concept of an AR agonist (e.g., SARM) combined with a CDK4/CDK6 inhibitor (e.g., palbociclib) can be viewed in Figure 1. As indicated in Figure 1, the SARM RAD140 has very effective tumor suppressing capability, similar to a monotherapy with palbociclib. Unexpectedly, a combination therapy of RAD140 and palbociclib provided enhanced TGI compared to monotherapy with RAD140 or palbociclib. Thus, a combination therapy of AR agonists and CDK4/6 inhibitors may provide improved activity and marked clinical benefit for treatment of AR+ breast cancer. Similarly, Figure 6 demonstrates efficacy alone or with palbociclib in the WHIM18 model initially expressing ESR1-YAP fusion where fulvestrant (as SERD) was ineffective alone and did not enhance the efficacy of palbociclib in the fulvestrant-palbociclib combination therapy.

### Administration and formulations

With regard to administration of the compound and combinations according to the invention, the AR agonist (i.e., Compound III) or solvates or salts thereof and the CDK4 and/or CDK6 inhibitors (e.g., ribociclib, abemaciclib and palbociclib) or the mTOR inhibitors, PI3K inhibitors, PARP inhibitors, MCL-1 inhibitors and/or BCL2 inhibitors disclosed herein are administered in combination to a subject in need. The phrase "in combination" means that the Compound III may be administered before, during, or after the administration of the CDK4 and/or CDK6 inhibitors or mTOR inhibitors, PI3K inhibitors, PARP inhibitors, MCL-1 inhibitors and/or BCL2 inhibitors. For example, the Compound III and the CDK4 and/or CDK6 inhibitor or mTOR inhibitor, PI3K inhibitors, PARP inhibitors, MCL-1 inhibitors and/or BCL2 inhibitors can be administered in about one week apart, about 6 days apart, about 5 days apart, about 4 days apart, about 3 days apart, about 2 days apart, about 24 hours apart, about 23 hours apart, about 22 hours apart, about 21 hours apart, about 20 hours apart, about 19 hours apart, about 18 hours apart, about 17 hours apart, about 16 hours apart, about 15 hours apart, about 14 hours apart, about 13 hours apart, about 12 hours apart, about 11 hours apart, about 10 hours apart, about 9 hours apart, about 8 hours apart, about 7 hours apart, about 6 hours apart, about 5 hours apart, about 4 hours apart, about 3 hours apart, about 2 hours apart, about 1 hour apart, about 55 minutes apart, about 50 minutes apart, about 45 minutes apart, about 40 minutes apart, about 35 minutes apart, about 30 minutes apart, about 25 minutes apart, about 20 minutes apart, about 15 minutes apart, about 10 minutes apart, or about 5 minutes apart. In certain embodiments, the Compound III and the CDK4 and/or CDK6 inhibitors or mTOR inhibitors, PI3K inhibitors, PARP inhibitors, MCL-1 inhibitors and/or BCL2 inhibitors are administered to the subject simultaneously or substantially simultaneously. In certain of these embodiments, the Compound III and the CDK4 and/or CDK6 inhibitor (e.g., ribociclib, abemaciclib and palbociclib) or mTOR inhibitors (e.g., sirolimus, temsirolimus, everolimus, ridafarolimus and MLN0128), PI3K inhibitors, PARP inhibitors, MCL-1 inhibitors and/or BCL2 inhibitors disclosed herein may be administered as part of a single formulation. Included are kits where Compound III and one or more of the additional agents described herein are contained within a kit together, for example as a copackaging arrangement. By way of non-limiting example, kits containing RAD140 with a CDK4/6 inhibitor, m-TOR inhibitor, PI3K inhibitor, PARP inhibitor, MCL-1 inhibitor and/or BCL2 inhibitor such as those detailed herein are included within the scope.

In some embodiments, the combination of Compound III and a single CDK4 and/or CDK6 inhibitor or mTOR inhibitor, PI3K inhibitor, PARP inhibitor, MCL-1 inhibitor and/or BCL2 inhibitor is administered to a subject. In certain embodiments, the combination of Compound III and a CDK4/CDK6 inhibitor and an mTOR inhibitor, PI3K inhibitor, PARP inhibitor, MCL-1 inhibitor and/or BCL2 inhibitor are administered together to a subject. Formulations of the Compound III used in the methods disclosed herein have been generally disclosed in the literature. In particular, US8,067,448 discloses how to make the compounds and general formulation methods useful for formulating compounds according to Formula I, Compound II and Compound III. Similarly, US9,133,182 discloses how to make the compounds according to Formula IV and formulate generally. In instances where specific formulation advice or direction is not available, some general principles to formulation may apply. For example, the compounds and combinations of the presently disclosed methods can be formulated into unit dosage forms, meaning physically discrete units suitable as unitary dosage for subjects undergoing treatment, with each unit containing a predetermined quantity of active material calculated to produce the desired therapeutic effect, optionally in association with a suitable pharmaceutical carrier. The unit dosage form can be for a single daily dose or one of multiple daily doses (e.g., about 1 to 4 or more times q.d.). When multiple daily doses are used, the unit dosage form can be the same or different for each dose. In certain embodiments, the compounds may be formulated for controlled release.

The compounds and combinations for use in the presently disclosed methods can be formulated according to any available conventional method. Examples of preferred dosage forms include a tablet, a powder, a subtle granule, a granule, a coated tablet, a capsule, a syrup, a troche, an inhalant, a suppository, an injectable, an ointment, an ophthalmic ointment, an eye drop, a nasal drop, an ear drop, a cataplasm, a lotion and the like. In the formulation, generally used additives such as a diluent, a binder, an disintegrant, a lubricant, a colorant, a flavoring agent, and if necessary, a stabilizer, an emulsifier, an absorption enhancer, a surfactant, a pH adjuster, an antiseptic, an antioxidant and the like can be used. In addition, the formulation is also carried out by combining compositions that are generally used as a raw material for pharmaceutical formulation, according to the conventional methods. Examples of these compositions include, for example, (1) an oil such as a soybean oil, a beef tallow and synthetic glyceride; (2) hydrocarbon such as liquid paraffin, squalane and solid paraffin; (3) ester oil such as octyldodecyl myristic acid and isopropyl myristic acid; (4) higher alcohol such as cetostearyl alcohol and behenyl alcohol; (5) a silicon resin; (6) a silicon oil; (7) a surfactant such as polyoxyethylene fatty acid ester, sorbitan fatty acid ester, glycerin fatty acid ester, polyoxyethylene sorbitan fatty acid ester, a solid polyoxyethylene castor oil and polyoxyethylene polyoxypropylene block co-polymer; (8) water soluble macromolecule such as hydroxyethyl cellulose, polyacrylic acid, carboxyvinyl polymer, polyethyleneglycol, polyvinylpyrrolidone and methylcellulose; (9) lower alcohol such as ethanol and isopropanol; (10) multivalent alcohol such as glycerin, propyleneglycol, dipropyleneglycol and sorbitol; (11) a sugar such as glucose and cane sugar; (12) an inorganic powder such as anhydrous silicic acid, aluminum magnesium silicicate and aluminum silicate; (13) purified water, and the like. Additives for use in the above formulations may include, for example, 1) lactose, corn starch, sucrose, glucose, mannitol, sorbitol, crystalline cellulose and silicon dioxide as the diluent; 2) polyvinyl alcohol, polyvinyl ether, methyl cellulose, ethyl cellulose, gum arabic, tragacanth, gelatine, shellac, hydroxypropyl cellulose, hydroxypropylmethyl cellulose, polyvinylpyrrolidone, polypropylene glycol-poly oxyethylene-block co-polymer, meglumine, calcium citrate, dextrin, pectin and the like as the binder; 3) starch, agar, gelatine powder, crystalline cellulose, calcium carbonate, sodium bicarbonate, calcium citrate, dextrin, pectic, carboxymethylcellulose/calcium and the like as the disintegrant; 4) magnesium stearate, talc, polyethyleneglycol, silica, condensed plant oil and the like as the lubricant; 5) any colorants whose addition is pharmaceutically acceptable is adequate as the colorant; 6) cocoa powder, menthol, aromatizer, peppermint oil, cinnamon powder as the flavoring agent; 7) antioxidants whose addition is pharmaceutically accepted such as ascorbic acid or alpha-tophenol.

The compounds and combinations for use in the invention can be formulated into a pharmaceutical composition as any one or more of the active compounds described herein and a physiologically acceptable carrier (also referred to as a pharmaceutically acceptable carrier or solution or diluent). Such carriers and solutions include pharmaceutically acceptable salts and solvates of compounds used in the methods of the instant invention, and mixtures comprising two or more of such compounds, pharmaceutically acceptable salts of the compounds and pharmaceutically acceptable solvates of the compounds. Such compositions are prepared in accordance with acceptable pharmaceutical procedures such as described in Remington's Pharmaceutical Sciences, 17th edition, ed. Alfonso R. Gennaro, Mack Publishing Company, Eaton, Pa. (1985).

The term "pharmaceutically acceptable carrier" refers to a carrier that does not cause an allergic reaction or other untoward effect in patients to whom it is administered and are compatible with the other ingredients in the formulation. Pharmaceutically acceptable carriers include, for example, pharmaceutical diluents, excipients or carriers suitably selected with respect to the intended form of administration, and consistent with conventional pharmaceutical practices. For example, solid carriers/diluents include, but are not limited to, a gum, a starch (e.g., corn starch, pregelatinized starch), a sugar (e.g., lactose, mannitol, sucrose, dextrose), a cellulosic material (e.g., microcrystalline cellulose), an acrylate (e.g., polymethylacrylate), calcium carbonate, magnesium oxide, talc, or mixtures thereof. Pharmaceutically acceptable carriers may further comprise minor amounts of auxiliary substances such as wetting or emulsifying agents, preservatives or buffers, which enhance the shelf life or effectiveness of the therapeutic agent.

AR agonists (e.g., SARMs) and/or CDK4/6 inhibitor and/or mTOR inhibitors, PI3K inhibitors, PARP inhibitors, MCL-1 inhibitors and/or BCL2 inhibitors in a free form can be converted into a salt, if need be, by conventional methods. The term "salt" used herein is not limited as long as the salt is pharmacologically acceptable; preferred examples of salts include a hydrohalide salt (for instance, hydrochloride, hydrobromide, hydroiodide and the like), an inorganic acid salt (for instance, sulfate, nitrate, perchlorate, phosphate, carbonate, bicarbonate and the like), an organic carboxylate salt (for instance, acetate salt, maleate salt, tartrate salt, fumarate salt, citrate salt and the like), an organic sulfonate salt (for instance, methanesulfonate salt, ethanesulfonate salt, benzenesulfonate salt, toluenesulfonate salt, camphorsulfonate salt and the like), an amino acid salt (for instance, aspartate salt, glutamate salt and the like), a quaternary ammonium salt, an alkaline metal salt (for instance, sodium salt, potassium salt and the like), an alkaline earth metal salt (magnesium salt, calcium salt and the like) and the like. In addition, hydrochloride salt, sulfate salt, methanesulfonate salt, acetate salt and the like are preferred as "pharmacologically acceptable salt" of the compounds disclosed herein.

In certain embodiments, the Compound III and CDK4/6 and/or mTOR inhibitors, PI3K inhibitors, PARP inhibitors, MCL-1 inhibitors and/or BCL2 inhibitors disclosed herein may be in a prodrug form, meaning that it must undergo some alteration (e.g., oxidation or hydrolysis) to achieve its active form.

The administration of the compounds and/or combinations disclosed herein can be by routes heretofore described for those compounds though in general such as transdermal, subcutaneously, intravenously, intranasally, pulmonary and oral. Oral is the preferred route for the combinations of this invention.

A therapeutically effective amount of a combination of an AR agonist (e.g., SARM) and CDK4/6 inhibitor and/or mTOR inhibitor, PI3K inhibitor, PARP inhibitor, MCL-1 inhibitor and/or BCL2 inhibitor in the methods disclosed herein is an amount that, when administered over a particular time interval, results in achievement of one or more therapeutic benchmarks (e.g., slowing or halting of tumor growth, resulting in tumor regression, cessation of symptoms, etc.). The combination for use in the presently disclosed methods may be administered to a subject one time or multiple times. In those embodiments wherein the compounds are administered multiple times, they may be administered at a set interval, e.g., daily, every other day, weekly, or monthly. Alternatively, they can be administered at an irregular interval, for example on an as-needed basis based on symptoms, patient health, and the like. A therapeutically effective amount of the combination may be administered q.d. for one day, at least 2 days, at least 3 days, at least 4 days, at least 5 days, at least 6 days, at least 7 days, at least 10 days, or at least 15 days. Optionally, the status of the cancer or the regression of the tumor is monitored during or after the treatment, for example, by a FES-PET scan of the subject. The dosage of the combination administered to the subject can be increased or decreased depending on the status of the cancer or the regression of the tumor detected.

The skilled artisan can readily determine this amount, on either an individual subject basis (e.g., the amount of a compound necessary to achieve a particular therapeutic benchmark in the subject being treated) or a population basis (e.g., the amount of a compound necessary to achieve a particular therapeutic benchmark in the average subject from a given population). Ideally, the therapeutically effective amount does not exceed the maximum tolerated dosage at which 50% or more of treated subjects experience nausea, hirsutism, voice hoarsening or other more serious reactions that prevent further drug administrations. A therapeutically effective amount may vary for a subject depending on a variety of factors, including variety and extent of the symptoms, sex, age, body weight, or general health of the subject, administration mode and salt or solvate type, variation in susceptibility to the drug, the specific type of the disease, and the like. One means of demonstrating acute response to the present treatment regimens is to analyze progestin receptor expression. It has been discovered that the Compound III used in the present invention leads to decreased expression of the progestin receptor indicating a response to the agent. Based on the extensive preclinical efficacy data in mouse xenografts presented in the examples, the calculation and disclosure of predicted effective human clinical doses for the SARM RAD140 is described in Example 9.

Examples of methods disclosed herein are for illustrative purposes and the invention is therefore not limited to the exemplified embodiments.

### Examples

### Example 1: SARM RAD140 in combination with CDK inhibitor inhibited ER and AR positive breast cancer growth in PDx mice(Figure 1)

### Material and Methods:

PDx model #1 maintained by a Contract Research Organization (CRO) was characterized as AR+/ER+/PR+/Her2- using IHC and gene chip microarray. Donor tumor slices were implanted subcutaneously into the flanks of intact female nude mice (n=7). Animals bearing tumors of sizes between 60-256 mm³ were randomized into four treatment groups. Animals of each group received vehicle (circle, Figure 1), RAD140 100 mg/kg twice a day (bid) (triangle, Figure 1), palbociclib 75 mg/kg once a day (qd) (square, Figure 1), or a combination of RAD140 100 mg/kg (bid) with palbociclib 75mg/kg (qd) (diamond, Figure 1), respectively. All test compounds were administered orally for 42 consecutive days. Tumor volume was measured twice weekly and % tumor growth inhibition (TGI) was calculated. Animals bearing tumors of size over 2,000 mm³ were euthanized per animal welfare regulation. At the end of the study, plasma and tumor samples were collected for analyses of pharmacokinetics and pharmacodynamics. The mice were supplemented with estradiol added to their water in order to stimulate the growth of the tumors.

### Results:

Treatment with RAD140 or palbociclib alone led to inhibition of tumor growth with approximately 53% TGI (tumor growth inhibition), respectively (Figure 1). The combined administration of SARM and CDK4/6 inhibitor produced a TGI of 70% on day 28, at which point the number of animals in the vehicle-treated group dropped below 6 due to ethical termination of mice with larger tumors. The combination of RAD140 and palbociclib continued to exhibit potent tumor suppressive effect until the end of the study (day 42). The estimated endpoint TGI was higher than 70%. No appreciable degree of RAD140-treatment related weight loss was observed (data not shown).

In summary, the treatment with a SARM alone exhibited tumor inhibition comparable to that with palbociclib in AR+/ER+/PR+/Her2- breast cancer PDx-bearing mice. The combined administration of RAD140 with palbociclib produced enhanced growth inhibitory effect in these xenografts than RAD140 and palbociclib effected alone respectively. These results indicate combined administration of a SARM with or without a CDK4/6 inhibitor is efficacious in ER+/AR+ mammary tumors, and that a combination with a CDK4/6 enhanced TGI more than the SARM and CKD4/6 inhibitor could when administered alone respectively.

### Example 2: Administration of SARM RAD140 in combination with a CDK inhibitor or an mTOR inhibitor inhibited ER and AR positive breast cancer growth in CDx mice (Figure 2)

### Material and Methods:

ZR-75-1 is a frequently used breast cancer cell line model that is ER+/AR+. The parental ZR-75-1 CDx model (ZR75 model) was established by injecting ZR-75-1 cells (donor tumors) into the flank of female nude mice. The ER+/AR+ status of these donor tumors were confirmed using immunoblotting (IB) and IHC. Donor tumor slices were implanted subcutaneously into the flanks of intact female nude mice (n=7). After randomnization of the established xenograft tumors, animals of each group received vehicle (diamond, Figure 2), RAD140 100 mg/kg (bid) (square, Figure 2), palbociclib 45 mg/kg (qd) (triangle, Figure 2), a combination of RAD140 100 mg/kg (bid) with palbociclib 45mg/kg (qd) (star, Figure 2), everolimus 2.5 mg/kg (qd) ("X," Figure 2), or a combination of RAD140 100 mg/kg (bid) with everolimus 2.5 mg/kg (qd) (circle, Figure 2), respectively. All test compounds were administered orally for 28 consecutive days. The ZR75-1 xenograft tumors in animals were supported by estradiol pellets (0.18 mg 90 day release, Innovative Research America). Tumor volume was measured twice weekly and %TGI was calculated. Animals bearing tumors of size over 2,000 mm³ were euthanized per animal welfare regulation. At the end of the study, plasma and tumor samples were collected for analyses of pharmacokinetics and pharmacodynamics.

### Results:

Treatment with RAD140, palbociclib or everolimus alone led to inhibition of tumor growth with TGIs of 52%, 84%, or 75%, respectively (Figure 2). The combination of RAD140 and palbociclib or everolimus exhibited potent tumor suppressive effect until the end of the study (day 28). The endpoint TGIs for RAD140-palbociclib combination and RAD140-everolimus combination were 96% and 101%, respectively. No appreciable degree of RAD140-treatment related weight loss was observed (data not shown).

In summary, the treatment with RAD140 alone exhibited anti-tumor activity in ER+/AR+ breast cancer CDx mice. The combined administration of RAD140 with either palbociclib or everolimus produced enhanced growth inhibitory effect in these xenografts than RAD140, palbociclib or everolimus alone, respectively. These results indicate a combined administration of the SARM RAD140 alone or in combination with either CDK4/6 inhibitor or mTOR is efficacious in ER+/AR+ mammary tumors.

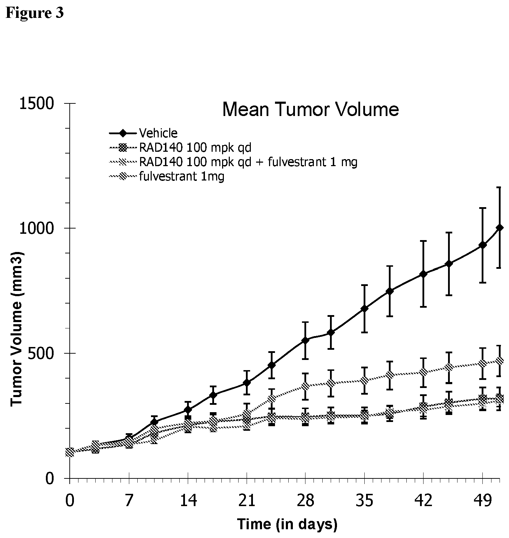

### Example 3: SARM reduced ER and AR breast cancer growth in PDx models and was more effective than fulvestrant (Figure 3)

### Material and Methods:

PDx model #2 maintained by a CRO was characterized as AR+/ER+ using IHC and gene chip microarray. Donor tumor slices were implanted subcutaneously into the flanks of intact female nude mice (n=10). After randomnization of the established xenografts, animals of each group received vehicle (diamond, Figure 3), RAD140 100 mg/kg daily (qd) (square, Figure 3), fulvestrant 1 mg weekly (qw) (circle, Figure 3), or a combination of RAD140 100 mg/kg (qd) with fulvestrant 1 mg (qw) (star, Figure 3), respectively. RAD140 was administered orally for 42 consecutive days and fulvestrant was administered subcutaneously once every week for 6 weeks. Tumor volume was measured twice weekly and %TGI was calculated. Animals bearing tumors of size over 2,000 mm³ were euthanized per animal welfare regulation. The PDx models were supplemented with estradiol added to their water to stimulate the growth of the tumors. At the end of the study, plasma and tumor samples were collected for analyses of pharmacokinetics and pharmacodynamics.

### Results:

Treatment with RAD140 alone led to inhibition of tumor growth with approximately 76% TGI (Figure 3). Fulvestrant alone led to 59% TGI. The combined administration of RAD140 and fulvestrant produced a TGI of 76%, similar to that observed with RAD140 alone. No appreciable degree of SARM-treatment related weight loss was observed (data not shown).

In summary, SARM alone exhibited more effective anti-tumor activity than a SERD (e.g., fulvestrant, a standard-of-care drug for ER+ breast cancers). Combined administration of SARM and fulvestrant did not show improvement in efficacy in ER+/AR+ breast cancer PDx mice beyond what RAD140 demonstrated alone.

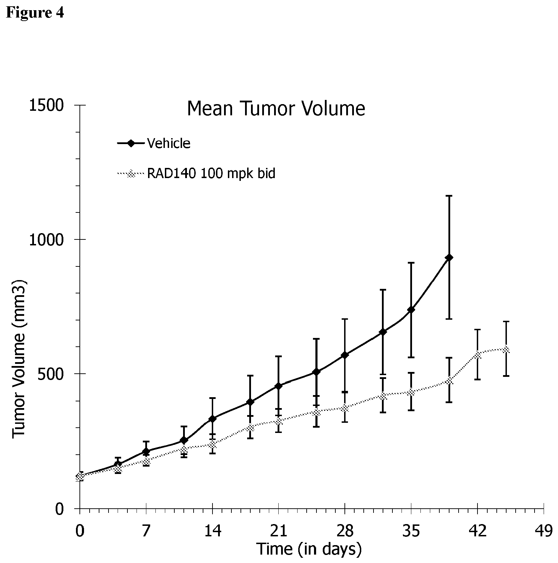

### Example 4: RAD140 reduced ER and AR positive breast cancer growth in PDx models (Figure 4)

### Material and Methods:

PDx model #3 maintained by a CRO was characterized as AR+/ER+ using IHC and gene chip microarray. Donor tumor slices were implanted subcutaneously into the flanks of intact female nude mice (n=10). After randomization of the established xenografts, animals of each group received vehicle (diamond, Figure 4) or RAD140 100 mg/kg (bid) (triangle, Figure 4) for 45 days. The PDx mice were supplemented with estradiol added to their water in order to stimulate the growth of the tumors.

### Results:

Treatment with RAD140 led to inhibition of tumor growth with 49% TGI (Figure 4). No appreciable degree of RAD140-treatment related weight loss was observed (data not shown).
In summary, these results indicates a SARM inhibits the growth of ER+/AR+ mammary tumors.

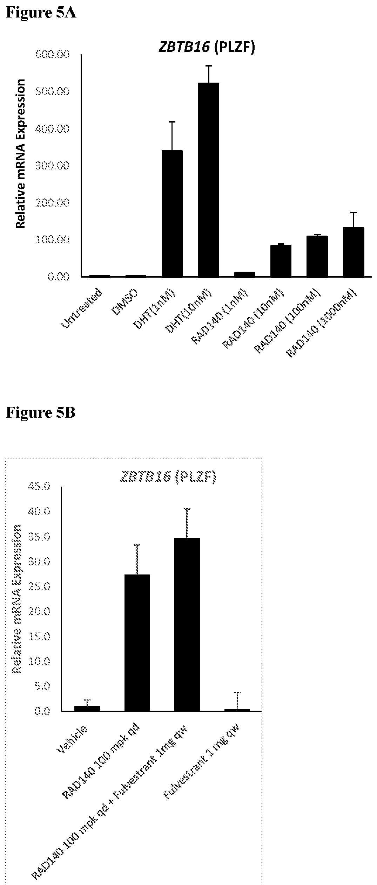

### Example 5: RAD140 induces AR target gene ZBTB16 expression in ER+/AR+ breast cancer cells and patient-derived xenograft (PDx) (Figure 5)

### Material and Methods:

PDX model #2 was treated as described in Example 3 and frozen tumor samples collected 6 h after the last dose. T47D breast cancer cells positive for ER and AR were incubated in media supplemented with 5% charcoal-dextran stripped serum (CSS) for 48 h before treatment with vehicle (DMSO), RAD140 at 1 nM, 10 nM, 100 nM, 1,000 nM or DHT at 1 nM or 10 nM. Twenty-four hours after treatment, cells were harvested. RNA was extracted from the frozen tumor samples from PDX #2 and T47D cells mentioned above using a Qiagen RNeasy kit. Real-time quantitative PCR (qPCR) was performed using primer/probe sets for the AR target gene *ZBTB16* (encoding PLZF protein) and *GAPDH* (internal control) (Applied Biosystems/ThermoScientific).

### Results:

Treatment of T47D cells *in vitro* with RAD140 led to a dose dependent increase in *ZBTB16* mRNA expression (Figure 5A), with the 1,000 nM treatment leading to about 130-fold increase compared to that in vehicle treated cells. The natural androgen DHT also led to 300-500-fold induction of this gene. This further supports that RAD140 is a potent AR agonist. Consistently, in PDX #2 treated with RAD140 100 mg/kg (mpk) qd for 45 days, a ~25-fold induction of *ZBTB16* gene was also seen compared to that in vehicle treated tumors (Figure 5B). In contrast, fulvestrant, a SERD and antagonist of ER pathway, did not lead to appreciable increase in *ZBTB16* gene expression (Figure 5B). *ZBTB16*/PLZF has been implicated in prostate cancer as a tumor suppressor that is suppressed in recurrent tumors after androgen-deprivation therapy (ADT, aka castration). These results suggest the SARM RAD140 activated the transcription of AR target gene in breast cancer cells. More importantly, RAD140 suppressed breast cancer growth by inducing certain tumor suppressor genes including but not limited to *ZBTB16*/PLZF.

### Example 6: RAD140 was effective in the WHIM18 PDx models with and without palbociclib

The WHIM18 PDx model is a patient derived xenograft of a breast cancer tumor transplanted into athymic female nude mice. The WHIM18 xenografts were ER+/PR+/AR+/HER- and grew independently of exogenous estrogen (e.g., 17β-estradiol). The WHIM18 PDx models were highly resistant to the potent ER-degrader fulvestrant. The WHIM18 PDx model harbored the ESR1-YAP1 fusion and an E545K mutation in PIK3CA.

The efficacy of RAD140 or fulvestrant alone and in combination with palbociclib in WHIM18 PDx models was evaluated for 56 days (8 weeks) of treatment. The primary endpoint was tumor growth. EDTA plasma and tumor tissues were collected after the last dose.

### Materials and Methods

Female Outbred Athymic Nude Mice (The Jackson Laboratory 007850) (DOB 7-19-16) were implanted with a single cells suspension of 1.5x10⁶ WHIM18 cells, passage 9. The cells were mixed 1:1 with DMEM:Matrigel (Corning REF 354234) in a total volume of 100µl/mouse. Once tumors reached a mean volume of approximately 100-300 mm³ (actual mean = 179.9), 60 mice were randomized by tumor volume into 1 of 6 treatment groups (10 mice/group) using Biopticon's TumorManager^{™} software on day 62 post implantation of cells.

Each mouse was dosed by a dosage regimen described in Table 1 for 56 days: vehicle 0.5% carboxymethylcellulose (CMC, Sigma C4888), RAD140, or palbociclib were dosed by oral gavage once per day; fulvestrant was dosed by subcutaneous injection once every seven days. The dose volumes were calculated based on average weekly animal weight for each group as shown in Table 1. Prepared 0.5% CMC by dissolving 2.5g in 400 ml warm sterile water, heat with stirring until dissolved, qs to 500 ml with sterile water stored at 4°C.

**Table 1: Dosage regimens of WHIM18 PDx models**

| Group # | Test Article | Dose | Dosing Route | Dosing Frequency |
|---|---|---|---|---|
| 1 (diamond, Figure 6) | Vehicle (0.5% CMC) | | po | qd |
| 2 (square, Figure 6) | RAD 140 | 100 mg/kg. | po | qd |
| 3 (triangle, Figure 6) | Fulvestrant | 250 mg/kg | sc | q7D |
| 4 ("X," Figure 6) | Palbociclib | 75 mg/kg | po | qd |
| 5 (star, Figure 6) | RAD 140 | 100 mg/kg | po | qd |
| | Palbociclib | 75 mg/kg | po | qd |
| 6 (circle, Figure 6) | Fulvestrant | 250 mg/kg | sc | q7D |
| | Palbociclib | 75 mg/kg | po | qd |

The RAD140 composition for administration in this example was prepared by adding an appropriate amount of 0.5% CMC to RAD140 with continuous stirring at 4 °C while protected from light. The RAD140 composition was prepared weekly.

The palbociclib composition for administration in this example was prepared by adding an appropriate amount of sterile saline (0.9% Sodium Chloride for injection NDC 0409-7983-03) to palbociclib with stirring. The palbociclib composition was continuously stirred at 4 °C until palbociclib dissolved. The palbociclib composition was prepared weekly.

The tumor volumes were measured twice per week using Biopticon's TumorImager^{™}, volumes were calculated using the corresponding TumorManager^{™} software. The mice were weighed once per week for the first half of the study (27 days). The mice were weighed twice per week once they showed weight loss. Additionally, any mouse with body weight loss (BWL) ≥ 5% compared to Day 0 or showed significant clinical signs (e.g., hunched posture, scruffy looking) was weighed daily. The dosing was suspended for any mice that had BWL ≥ 15% compared to Day 0 ; and the dosing was resumed when the body weight of the mice restored to BWL ≤ 15%.

The mice were removed from the study if they became moribund, if their tumor volume exceeded 2,000 mm³, or if they lost ≥ 20% of their body weight compared to Day 0. If possible, end of study samples were taken and the time of the last dose and take down time was recorded.

The remaining animals were taken down on day 56 approximately 6 hours following the last dose. The last dose of fulvestrant was given the morning of the takedown. The actual takedown was between 6-9 hours. Blood was collected via cardiac puncture immediately upon death via CO₂ and placed in EDTA tubes spun down at 2000 g for 10 minutes at 4 °C. The plasma was transferred to Eppendorf tube and stored at -80°C.

Following the blood collection the tumors were excised and weighed. For each tumor, half of the tumor was placed in 10% Neutral Buffered Formalin (NBF), and the other half of the tumor was placed in an Eppendorf tube, flash frozen in liquid N₂, and then stored at -80°C. All tissue collected for formalin-fixed paraffin-embedded (FFPE) blocks were fixed in neutral buffered formalin (NBF) for 24-48 hours and then transferred to 70% ethanol before being shipped to be processed into FFPE blocks.

### Results:

The results of the *in vivo* experiment are shown graphically in Figure 6. The relative reductions in tumor proliferation are shown with statistics below in Tables 2 and 3.

Tumor Growth Inhibition (TGI, in %) was calculated relative to the vehicle group on day 56 (Table 2, Formulation 1) $100 ∗ \left(1-\left(\frac{\left(Treatment Final Mean Volume-Treatment Baseline Mean Volume\right)}{\left(Vehicle Final Mean Volume-Vehicle Baseline Mean Volume\right)}\right)\right)$ Formulation 1

**Table 2: %TGI of WHIM18 PDx models treated with RAD140, fulvestrant, palbociclib, RAD140 + Palbociclib and Fulvestrant + Palbociclib**

| Group # | Test Article | %TGI |
|---|---|---|
| 1 (diamond, Figure 6) | Vehicle (0.5% CMC) | --- |
| 2 (square, Figure 6) | RAD 140 | 43.4 |
| 3 (triangle, Figure 6) | Fulvestrant | 1.2 |
| 4 ("X," Figure 6) | Palbociclib | 72.8 |
| 5 (star, Figure 6) | RAD140 + Palbociclib | 94.1 |
| 6 (circle, Figure 6) | Fulvestrant + Palbociclib | 77.8 |

Student's t-Test was calculated in excel using two-tailed distribution and two-sample equal variance of the delta tumor volume Day 56 - Day 0.

**Table 3: p values calculated from Student's t-Test of %TGI of WHIM18 PDx models treated with RAD140, fulvestrant, palbociclib, RAD140 + Palbociclib and Fulvestrant + Palbociclib**

| Test Articles | p value |
|---|---|
| RAD140 vs Vehicle | 0.070 |
| Palbociclib vs Vehicle | 0.002 |
| Fulvestrant vs Vehicle | 0.933 |
| RAD140 vs Palbociclib | 0.165 . |
| Fulvestrant + Palbociclib vs Vehicle | 0.001 |
| Fulvestrant + Palbociclib vs Fulvestrant | 0.004 |
| Fulvestrant + Palbociclib vs Palbociclib | 0.798 |
| RAD140 + Palbociclib vs Vehicle | 0.00001 |
| RAD140 + Palbociclib vs RAD140 | 0.006 |
| RAD140 + Palbociclib vs Palbociclib | 0.129 |

Administration of RAD140 at a dose of 100 mg/kg alone or in combination with palbociclib inhibited the growth of HER2-, ER+, PR+ breast cancer tumors implanted in female athymic nude mice (WHIM18 PDx) (Figure 6). The combination of RAD140 with palbociclib appears to be more effective than either drug alone. No apparent toxicity was observed in any of the groups.

### Example 7

In PDx model #2, i.e. the same PDx model as described in Example/Figure 3, RAD140 administered at lower doses, 1 mg/kg bid, or 10 mg/kg bid both led to substantial inhibition of tumor growth, as judged by TGI values of ~81% in both groups.

### Example 8

In PDx model #1, i.e. the same PDx model as described in Example/Figure 1, RAD140 administered at lower doses, 1 mg/kg bid, 3 mg/kg bid or 10 mg/kg bid all led to substantial inhibition of tumor growth, as judged by TGI values of 49%, 65% and 57%, respectively.

### Example 9

RAD140 was demonstrated to have good activity in a transplanted PDx tumor xenograft in nude mice. The activity was significant in a range from 1mg/kg through 100 mg/kg. Taking into account the specific exposure levels in mice, and cross species pharmacokinetic modelling from both known and derived pharmacokinetic parameters, a dose range in women patients can be calculated. In particular, doses between 1 mg/kg (bid) and 100 mg/kg (bid) were all demonstrated to have good efficacy in one or more models described in the examples herein. Based on half life across speies and microsome stability,it is predicted that RAD140 will be effective as a once per day oral dosage with a dose range between 5 mg and 500 mg. For example, it is believed that the mouse efficacious dose of 10 mg/kg (qd) effectively translates to a dose of approximately 50 mg qd in a 60kg woman. Since a range of 1 mg/kg to 100 mg/kg (bid) were shown effective, a broader range of 10 mg to 1000 mg is clinically relevant. In particular, within this range it can be seen that additional ranges of 10 mg - 250 mg, 25 mg - 250 mg, 25-500 are also supported. Similarly, individual dose points falling anywhere within the range are well supported such that any specific point within the range, integer or non-integer are supported. For example, doses like 12.5 mg, 17.5 mg and so on are specifically contemplated as are doses such as 10 mg, 25 mg, 50 mg, 75 mg, 100 mg, 150 mg, 200 mg, 300 mg, 400 mg, and 500 mg. By way of further non-limited examples, a dose of 10 mg, 15 mg, 20 mg, 25 mg, 50 mg, 75, 100, 125, 150, 175, 200, 250, 300, 350, 400, 450, 500 mg. QD dosing of the described doses are predicted to be quite adequate as RAD140 is predicted from pharmacokinetic studies in animals to have a long half life suitable for once daily dosing though bid dosing would also work BUT the doses given above for a single daily administration are divided in two since they would be given twice per day.

## Claims

1. A compound which is Compound III: 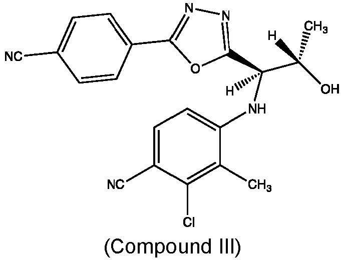 a pharmaceutically acceptable salt thereof, or a pharmaceutically acceptable solvate thereof, for use in a method of treating AR+/ER+/HER2- breast cancer in a subject.

2. The compound for use according to claim 1, wherein the compound is administered via an oral route.

3. The compound for use according to claim 1 or 2 wherein:
(i) the subject is treated in an adjuvant setting;
(ii) the subject has had disease progression after treatment with one or more endocrinological agents; optionally wherein said one or more endocrinological agents are selected from the group consisting of SERMs, SERDs, progestins, aromatase inhibitors, and combinations thereof; or
(iii) said subject has had disease progression after treatment with one or more agents selected from the group consisting of CDK4/6 inhibitors, mTOR inhibitors, BCL-2 inhibitors, PI3K inhibitors, and combinations thereof.

4. The compound for use according to any preceding claim, wherein:
(i) the breast cancer is resistant to at least one prior therapy; and/or
(ii) the subject has progressed on prior endocrine therapy.

5. The compound for use according to claim 1, wherein the subject is a candidate for first line treatment when the subject expresses an ER mutation or fusion.

6. The compound for use according to any preceding claim, wherein the compound is dosed between 10 and 500 mg, 10 mg and 250 mg, or 25 mg and 250 mg per day; optionally wherein the dose is once per day.

7. The compound for use according to any preceding claim, wherein the subject expresses:
(i) ESR1 comprising one or more mutations, optionally wherein said mutation comprises one or more mutations selected from the group consisting of ESR1-AKAP12, ESR1-CCDC170, ESR1-YAP1, ESR1-POLH, ESR1-PCDH11X, and combinations thereof;
(ii) a PI3K mutation; and/or
(iii) an E545K mutation in PIK3CA.

8. The compound for use according to any preceding claim, wherein the treatment further comprises the administration of a CDK4/6 inhibitor, optionally, wherein said CDK4/6 inhibitor is selected from the group consisting of palbociclib, ribociclib, trilaciclib, abemaciclib and AMG925.

9. The compound for use according to any preceding claim, wherein said treatment further comprises the administration of a second therapeutic agent that targets the PI3K/AKT/mTOR pathway, for example:
(i) an mTOR inhibitor; optionally wherein said mTOR inhibitor is selected from the group consisting of sirolimus, temsirolimus, everolimus, ridafarolimus, and MLN0128; and/or
(ii) a PI3K inhibitor; optionally wherein said PI3K inhibitor is BEZ235, GDC-0980, GDC-0941, BYL719, MK2206, GDC-0068, GSK2110183, GSK2141795, AZD5363, AZD2014, MLN0128 or CC-223.

10. The compound for use according to any preceding claim, wherein said treatment further comprises the administration of:
(i) a PARP inhibitor, for example talazoparib, veliparib, niraparib, beigene290, E7449, KX01, ABT767, CK102, JPI289, KX02, IMP4297, SC10914, NT125, PJ34, VPI289 or ANG-3186;
(ii) a MCL-1 inhibitor; optionally wherein said MCL-1 inhibitor is 7-(5-((4-(4-(N,N-Dimethylsulfamoyl)piperazin-1-yl)phenoxy)methyl)-1,3-dimethyl-1H-pyrazol-4-yl)-1-(2-morpholinoethyl)-3-(3-(naphthalen-1-yloxy)propyl)-1H-indole-2-carboxylic Acid, S63845, omacataxine, seliciclib, UMI-77, AT101, or sabutoclax; and/or
(iii) a BCL-2 inhibitor, for example venetoclax, navitoclax, ABT737, G3139 or S55746.

11. The compound for use according to any one of claims 1 to 8, wherein said treatment further comprises the administration of a therapeutic agent selected from:
(i) rapamycin, everolimus, ridaforolimus, or temsirolimus;
(ii) a mTORC1 and mTOR2 inhibitor selected from the group consisting of MLN0128, CC115, CC223, OSI-027, AZD8055, AZD2014; or
(iii) a PI3K/mTOR inhibitor selected from the group consisting of GDC-0980, SAR245409, LY3023414, NVP-BEZ235, NVP-BGT226, SF1126 and PKI-587.

12. The compound for use according to any one of claims 1 to 7, wherein the treatment further comprises administration of a therapeutic agent selected from:
(i) a mTOR inhibitor that is everolimus;
(ii) a PI3K inhibitor selected from the group consisting of BYL719, GDC-0068, AZD5363; or
(iii) a CDK4/6 inhibitor selected from the group consisting of palbociclib, and abemaciclib.

13. The compound for use according to claim 1 to 8, wherein the treatment further comprises the administration of:
(i) a mTOR inhibitor that is everolimus; or
(ii) a PI3K inhibitor that is BYL719.

14. The compound for use according to any preceding claim, wherein:
(i) said subject is a woman, optionally wherein said woman is a premenopausal woman or a post-menopausal woman; and/or
(ii) said breast cancer is localized, advanced or metastatic.

## Patentansprüche

1. Verbindung, bei der es sich um Verbindung III: 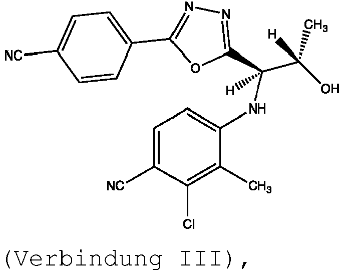 ein pharmazeutisch unbedenkliches Salz davon oder ein pharmazeutisch unbedenkliches Solvat davon handelt, zur Verwendung bei einem Verfahren zur Behandlung von AR+/ER+/HER2--Brustkrebs bei einem Individuum.

2. Verbindung zur Verwendung nach Anspruch 1, wobei die Verbindung über einen oralen Weg verabreicht wird.

3. Verbindung zur Verwendung nach Anspruch 1 oder 2, wobei:
(i) das Individuum im Rahmen einer adjuvanten Therapie behandelt wird;
(ii) die Krankheit bei dem Individuum nach Behandlung mit einem oder mehreren endokrinologischen Mitteln fortgeschritten war; gegebenenfalls wobei die ein oder mehreren endokrinologischen Mittel aus der Gruppe bestehend aus SERMs, SERDs, Progestinen, Aromatase-Inhibitoren und Kombinationen davon ausgewählt sind; oder
(iii) die Krankheit bei dem Individuum nach Behandlung mit einem oder mehreren Mitteln, die aus der Gruppe bestehend aus CDK4/6-Inhibitoren, mTOR-Inhibitoren, BCL-2-Inhibitoren, PI3K-Inhibitoren und Kombinationen davon ausgewählt sind, fortgeschritten war.

4. Verbindung zur Verwendung nach einem vorhergehenden Anspruch, wobei:
(i) der Brustkrebs gegenüber mindestens einer früheren Therapie resistent ist; und/oder
(ii) bei dem Individuum auf eine frühere endokrine Therapie ein Fortschreiten eingetreten ist.

5. Verbindung zur Verwendung nach Anspruch 1, wobei das Individuum ein Kandidat für eine Erstlinienbehandlung ist, wenn bei dem Individuum die Expression einer ER-Mutation oder -Fusion vorliegt.

6. Verbindung zur Verwendung nach einem vorhergehenden Anspruch, wobei die Verbindung in einer Dosis zwischen 10 und 500 mg, 10 mg und 250 mg oder 25 mg und 250 mg pro Tag gegeben wird; gegebenenfalls wobei die Gabe einmal pro Tag erfolgt.

7. Verbindung zur Verwendung nach einem vorhergehenden Anspruch, wobei bei dem Individuum die Expression von Folgendem vorliegt:
(i) ESR1, umfassend eine oder mehrere Mutationen, gegebenenfalls wobei die Mutation eine oder mehrere Mutationen umfasst, die aus der Gruppe bestehend aus ESR1-AKAP12, ESR1-CCDC170, ESR1-YAP1, ESR1-POLH, ESR1-PCDH11X und Kombinationen davon ausgewählt sind;
(ii) eine PI3K-Mutation; und/oder
(iii) eine E545K-Mutation in PIK3CA.

8. Verbindung zur Verwendung nach einem vorhergehenden Anspruch, wobei die Behandlung ferner die Verabreichung eines CDK4/6-Inhibitors umfasst, gegebenenfalls wobei der CDK4/6-Inhibitor aus der Gruppe bestehend aus Palbociclib, Ribociclib, Trilaciclib, Abemaciclib und AMG925 ausgewählt ist.

9. Verbindung zur Verwendung nach einem vorhergehenden Anspruch, wobei die Behandlung ferner die Verabreichung eines zweiten Therapeutikums umfasst, das auf den PI3K/AKT/mTOR-Weg zielt, zum Beispiel:
(i) ein mTOR-Inhibitor; gegebenenfalls wobei der mTOR-Inhibitor aus der Gruppe bestehend aus Sirolimus, Temsirolimus, Everolimus, Ridafarolimus und MLN0128 ausgewählt ist; und/oder
(ii) ein PI3K-Inhibitor; gegebenenfalls wobei es sich bei dem PI3K-Inhibitor um BEZ235, GDC-0980, GDC-0941, BYL719, MK2206, GDC-0068, GSK2110183, GSK2141795, AZD5363, AZD2014, MLN0128 oder CC-223 handelt.

10. Verbindung zur Verwendung nach einem vorhergehenden Anspruch, wobei die Behandlung ferner die Verabreichung:
(i) eines PARP-Inhibitors, zum Beispiel Talazoparib, Veliparib, Niraparib, beigene290, E7449, KX01, ABT767, CK102, JPI289, KX02, IMP4297, SC10914, NT125, PJ34, VPI289 oder ANG-3186, umfasst;
(ii) eines MCL-1-Inhibitors umfasst; gegebenenfalls wobei es sich bei dem MCL-1-Inhibitor um 7-(5-((4-(4-(N,N-Dimethylsulfamoyl)piperazin-1-yl)phenoxy)methyl)-1,3-dimethyl-1H-pyrazol-4-yl)-1-(2-morpholinoethyl)-3-(3-(naphthalen-1-yloxy)propyl)-1H-indol-2-carbonsäure, S63845, Omacataxin, Seliciclib, UMI-77, AT101 oder Sabutoclax handelt; und/oder
(iii) eines BCL-2-Inhibitors, zum Beispiel Venetoclax, Navitoclax, ABT737, G3139 oder S55746, umfasst.

11. Verbindung zur Verwendung nach einem der Ansprüche 1 bis 8, wobei die Behandlung ferner die Verabreichung eines Therapeutikums umfasst, das ausgewählt ist aus:
(i) Rapamycin, Everolimus, Ridaforolimus oder Temsirolimus;
(ii) einem mTORC1- und mTOR2-Inhibitor, der aus der Gruppe bestehend aus MLN0128, CC115, CC223, OSI-027, AZD8055, AZD2014 ausgewählt ist; oder
(iii) einem PI3K/mTOR-Inhibitor, der aus der Gruppe bestehend aus GDC-0980, SAR245409, LY3023414, NVP-BEZ235, NVP-BGT226, SF1126 und PKI-587 ausgewählt ist.

12. Verbindung zur Verwendung nach einem der Ansprüche 1 bis 7, wobei die Behandlung ferner die Verabreichung eines Therapeutikums umfasst, das ausgewählt ist aus:
(i) einem mTOR-Inhibitor, bei dem es sich um Everolimus handelt;
(ii) einem PI3K-Inhibitor, der aus der Gruppe bestehend aus BYL719, GDC-0068, AZD5363 ausgewählt ist; oder
(iii) einem CDK4/6-Inhibitor, der aus der Gruppe bestehend aus Palbociclib und Abemaciclib ausgewählt ist.

13. Verbindung zur Verwendung nach Anspruch 1 bis 8, wobei die Behandlung ferner die Verabreichung:
(i) eines mTOR-Inhibitors umfasst, bei dem es sich um Everolimus handelt; oder
(ii) eines PI3K-Inhibitors umfasst, bei dem es sich um BYL719 handelt.

14. Verbindung zur Verwendung nach einem vorhergehenden Anspruch, wobei:
(i) es sich bei dem Individuum um eine Frau handelt, gegebenenfalls wobei es sich bei der Frau um eine prämenopausale Frau oder eine postmenopausale Frau handelt; und/oder
(ii) der Brustkrebs lokalisiert, fortgeschritten oder metastatisch ist.

## Revendications

1. Composé qui est le composé III : 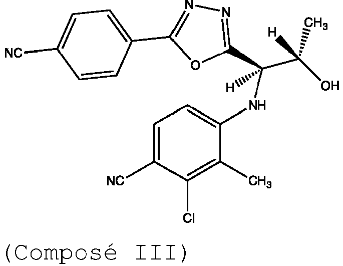 un sel pharmaceutiquement acceptable de celui-ci, ou un solvate pharmaceutiquement acceptable de celui-ci, destiné à être utilisé dans un procédé de traitement du cancer du sein AR+/ER+/HER2- chez un sujet.

2. Composé pour une utilisation selon la revendication 1, dans lequel le composé est administré par une voie orale.

3. Composé pour une utilisation selon la revendication 1 ou 2, dans lequel :
(i) le sujet est traité en situation adjuvante ;
(ii) le sujet a eu une évolution de la maladie après un traitement par un ou plusieurs agents endocrinologiques ; facultativement dans lequel lesdits un ou plusieurs agents endocrinologiques sont choisis dans le groupe constitué par des SERM, des SERD, des progestatifs, des inhibiteurs d'aromatase, et des combinaisons de ceux-ci ; ou
(iii) ledit sujet a eu une évolution de la maladie après traitement par un ou plusieurs agents choisis dans le groupe constitué par les inhibiteurs de CDK4/6, les inhibiteurs de mTOR, les inhibiteurs de BCL-2, les inhibiteurs de PI3K, et des combinaisons de ceux-ci.

4. Composé pour une utilisation selon une quelconque revendication précédente, dans lequel :
(i) le cancer du sein est résistant à au moins une thérapie précédente ; et/ou
(ii) le sujet a progressé sous une thérapie endocrinienne précédente.

5. Composé pour une utilisation selon la revendication 1, dans lequel le sujet est un candidat pour un traitement de première intention lorsque le sujet exprime une mutation ou fusion ER.

6. Composé pour une utilisation selon l'une quelconque des revendications précédentes, dans lequel le composé est administré en une quantité entre 10 et 500 mg, 10 mg et 250 mg, ou 25 mg et 250 mg par jour ; facultativement, dans lequel la dose est une fois par jour.

7. Composé pour une utilisation selon l'une quelconque des revendications précédentes, dans lequel le sujet exprime :
(i) ESR1 comprenant une ou plusieurs mutations, facultativement dans lequel ladite mutation comprend une ou plusieurs mutations choisies dans le groupe constitué par ESR1-AKAP12, ESR1-CCDC170, ESR1-YAP1, ESR1-POLH, ESR1-PCDH11X, et leurs combinaisons ;
(ii) une mutation PI3K ; et/ou
(iii) une mutation E545K dans PIK3CA.

8. Composé pour une utilisation selon l'une quelconque des revendications précédentes, dans lequel le traitement comprend en outre l'administration d'un inhibiteur de CDK4/6, facultativement, dans lequel ledit inhibiteur de CDK4/6 est choisi dans le groupe constitué par palbociclib, ribociclib, trilaciclib, abemaciclib et AMG925.

9. Composé pour une utilisation selon l'une quelconque des revendications précédentes, dans lequel ledit traitement comprend en outre l'administration d'un deuxième agent thérapeutique qui cible la voie PI3K/AKT/mTOR, par exemple :
(i) un inhibiteur de mTOR ; facultativement dans lequel ledit inhibiteur de mTOR est choisi dans le groupe constitué par le sirolimus, le temsirolimus, l'évérolimus, le ridafarolimus et le MLN0128 ; et/ou
(ii) un inhibiteur de PI3K ; facultativement dans lequel ledit inhibiteur de PI3K est BEZ235, GDC-0980, GDC-0941, BYL719, MK2206, GDC-0068, GSK2110183, GSK2141795, AZD5363, AZD2014, MLN0128 ou CC-223.

10. Composé pour une utilisation selon l'une quelconque des revendications précédentes, dans lequel ledit traitement comprend en outre l'administration de :
(i) un inhibiteur de PARP, par exemple le talazoparib, le véliparib, le niraparib, le béigène290, E7449, KX01, ABT767, CK102, JPI289, KX02, IMP4297, SC10914, NT125, PJ34, VPI289 ou ANG-3186 ;
(ii) un inhibiteur de MCL-1 ; facultativement dans lequel ledit inhibiteur de MCL-1 est l'acide 7-(5-((4-(4-(N,N-diméthylsulfamoyl)pipérazin-1-yl)phénoxy)méthyl)-1,3-diméthyl-1H-pyrazol-4-yl)-1-(2-morpholinoéthyl)-3-(3-(naphtalén-1-yloxy)propyl)-1H-indole-2-carboxylique, S63845, omacataxine, séliciclib, UMI-77, AT101 ou sabutoclax ; et/ou
(iii) un inhibiteur de BCL-2, par exemple le vénétoclax, le navitoclax, ABT737, G3139 ou S55746.

11. Composé pour une utilisation selon l'une quelconque des revendications 1 à 8, dans lequel ledit traitement comprend en outre l'administration d'un agent thérapeutique choisi parmi :
(i) rapamycine, évérolimus, ridaforolimus ou temsirolimus ;
(ii) un inhibiteur de mTORC1 et mTOR2 choisi dans le groupe constitué par MLN0128, CC115, CC223, OSI-027, AZD8055, AZD2014 ; ou
(iii) un inhibiteur de PI3K/mTOR choisi dans le groupe constitué par GDC-0980, SAR245409, LY3023414, NVP-BEZ235, NVP-BGT226, SF1126 et PKI-587.

12. Composé pour une utilisation selon l'une quelconque des revendications 1 à 7, dans lequel le traitement comprend en outre l'administration d'un agent thérapeutique choisi parmi :
(i) un inhibiteur de mTOR qui est l'évérolimus ;
(ii) un inhibiteur de PI3K choisi dans le groupe constitué par BYL719, GDC-0068, AZD5363 ; ou
(iii) un inhibiteur de CDK4/6 choisi dans le groupe constitué par le palbociclib et l'abémaciclib.

13. Composé pour une utilisation selon la revendication 1 à 8, dans lequel le traitement comprend en outre l'administration de :
(i) un inhibiteur de mTOR qui est l'évérolimus ; ou
(ii) un inhibiteur de PI3K qui est BYL719.

14. Composé pour une utilisation selon une quelconque revendication précédente, dans lequel :
(i) ledit sujet est une femme, facultativement, ladite femme étant une femme pré-ménopause ou une femme postménopause ; et/ou
(ii) ledit cancer du sein est localisé, avancé ou métastasique.
